# EUROPÄISCHE PATENTSCHRIFT

(11) **EP 3 004 321 B1**
(45) Veröffentlichungstag und Bekanntmachung des Hinweises auf die Patenterteilung: **09.10.2019**
(21) Anmeldenummer: 14728544.9
(22) Anmeldetag: 04.06.2014
(51) Int. Cl.: C12M 1/107, C12M 1/42, C12M 1/00, C12N 13/00

(54) **VORRICHTUNG ZUR ELEKTRISCHEN DESINTEGRATION VON ZELLVERBÄNDEN**
DEVICE FOR ELECTRICALLY DISINTEGRATING CELL CLUSTERS
DISPOSITIF DE DÉSINTÉGRATION ÉLECTRIQUE DE GROUPES DE CELLULES

(30) Priorität: 04.06.2013 DE 202013005125 U
(43) Veröffentlichungstag der Anmeldung: 13.04.2016
(73) Patentinhaber: Hugo Vogelsang Maschinenbau GmbH, 49632 Essen (DE); Prometheus GmbH & Co. KG, 49624 Löningen-Bunnen (DE)
(72) Erfinder: FLERLAGE, Paul, 49624 Löningen-Bunnen (DE)
(74) Vertreter: Eisenführ Speiser
(86) Internationale Anmeldenummer: PCT/EP2014/061548
(87) Internationale Veröffentlichungsnummer: WO 2014/195343

(56) Entgegenhaltungen:
- WO-A1-2012/000056
- WO-A2-03/060114
- DE-A1- 3 538 194
- DE-A1- 19 757 793
- DE-T2- 69 033 273
- DE-U1-202011 004 177
- US-A- 4 822 470

## Beschreibung

Die vorliegende Erfindung betrifft eine Vorrichtung zur elektrischen Desintegration von Zellverbänden, mit einer Elektrodeneinheit, welche einen Elektrodenkopf und einen Elektrodenkörper aufweist; einer Kammer, innerhalb welcher der Elektrodenkörper angeordnet ist, wobei die Kammer eine Wand aufweist, die abschnittsweise oder vollständig elektrisch leitfähig und von dem Elektrodenkörper elektrisch isoliert ist, und wobei die Kammer einen Einlass zur Aufnahme von Zellverbände enthaltendem Fluid aufweist, einer in dem Elektrodenkopf angeordneten Hochspannungsquelle, die angepasst ist, um mittels Anlegen einer elektrischen Spannung zwischen dem Elektrodenkörper und der Wand ein elektrisches Feld zu erzeugen, und einer elektronischen Steuereinheit, die mit der Hochspannungsquelle zusammenwirkt, um das elektrische Feld zu ändern.

Eine derartige Vorrichtung ist aus DE 20 2011 004 177 U1 der hiesigen Anmelderin bekannt. Solche Vorrichtungen werden in unterschiedlichen Bereichen eingesetzt, vornehmlich zur Behandlung von Fluidgemischen mit organischer Materie, insbesondere Zellen und/oder Zellverbände enthaltend, in Biogasanlagen und Kläranlagen. Ziel ist es, mittels der Desintegration der Zellverbände beispielsweise bei Biogasanlagen die Erzeugung von Biogas zu begünstigen, weil durch das sogenannte Cracken der Zellverbände die Reaktion von Ausgangsstoffen zu Faulgas begünstigt wird. Unter dem Begriff der Desintegration wird allgemein die Zerkleinerung von Zellen oder Zellverbänden unter Einwirkung äußerer Kräfte verstanden.

Andere bekannte Desintegrationsmethoden sind die thermische Desintegration, die Ultraschalldesintegration, chemische Desintegration und die mechanische Desintegration.

Die elektrische Desintegration beruht auf dem Funktionsprinzip, Zellverbände einem elektrischen Feld auszusetzen, welches zwischen zwei Elektroden anliegt. Infolge der Einwirkung des elektrischen Feldes auf die Zellen und Zellverbände kommt es an den Zellmembranen zu Ladungsverschiebungen. Bei den bekannten Anlagen zur elektrischen Desintegration wird hierbei sodann ausgenutzt, dass die Zellen und Zellverbände sich innerhalb der Kammer, in der das elektrische Feld anliegt, bewegen. Durch die Bewegung der Zellen und Zellverbände ändert sich im Bezug auf deren jeweilige Zellmembrane lokal die sie beeinflussende Feldstärke. Durch diese andauernde Änderung wird die Zellmembran und/oder der Zellverband Scherkräften und Vibrationen ausgesetzt, was zu einer Destabilisierung führt. Bei ausreichend starker Anregung wird der Zellverband aufgelockert oder aufgelöst. Bei stärkerer Beeinflussung werden die Zellmembranen kollabiert. Letzteres ist unter dem Begriff der Elektroporation bekannt. Der Effekt dieser Desintegration ist jener, dass die Verfügbarkeit insbesondere von Nährstoffen für fermentierende Bakterien deutlich erhöht wird. Dieser Effekt wird in Biogasanlagen vorteilhaft dazu genutzt, eine gesteigerte Gasausbeute und bessere Nutzung der dort angesetzten Substrate zu erreichen. Anlagen unter Nutzung der elektrischen Desintegration und des Verfahrens zur elektrischen Desintegration selbst sind den alternativen Desintegrations-Verfahren im Bezug auf die aufzuwendenden Investitionskosten, die Energieaufnahme und den apparativen Aufwand überlegen.

In der eingangs genannten DE 20 2011 004 177 U1 wird vorgeschlagen die Desintegrationsleistung dadurch zu erhöhen, dass eine Steuereinheit mit der Hochspannungsquelle zusammenwirkt, um das elektrische Feld zu ändern, wobei die Steuereinheit dazu eingerichtet ist, die Spannung zwischen der Elektrode und der Wand zu verändern. Es hat sich herausgestellt, dass die Änderung des elektrischen Feldes zu einer deutlichen Leistungssteigerung bei der Desintegration führt. Die Desintegrationsleistung und damit auch die mögliche Steigerung mittels der in DE 20 2011 004 177 U1 vorgeschlagenen Vorrichtung ist allerdings abhängig von den jeweils zur Gaserzeugung verwendeten Fluidgemischen mit organischer Materie, also ob beispielsweise nachwachsende Rohstoffe oder etwa Schlachtabfälle genutzt werden, sowie von dem schon erreichten Desintegrationsgrad der organischen Materie. Daher besteht ein Bedarf, die Leistungssteigerung der Desintegration dahingehend zu optimieren, dass die Vorrichtung zur elektrischen Desintegration von Zellverbänden rasch an sich ändernde Umgebungsbedingungen, insbesondere rasch an geänderte Eigenschaften der organischen Materie angepasst werden kann, und zwar derart, dass bei gegebener Eingangsspannung für die Vorrichtung ein möglichst starkes elektrisches Feld in der Kammer für die Desintegration zur Verfügung steht.

Bei den Vorrichtungen gemäß dem Stand der Technik hat sich allerdings gezeigt, dass es aufgrund des konstruktiven Aufbaus, beispielsweise aufgrund einer Erdung der Kammer, nicht ohne weiteres möglich ist, das elektrische Feld im Kammer-Inneren direkt zu messen, so dass man auf im Wege der Kalibrierung vorbestimmte Betriebsparameter der Anlage für die jeweils zu erwartenden Fluide angewiesen war.

Aus WO 2012/000056 A1 ist ein System zum Ernten von Biomasse-Mikroorganismen aus einer wässrigen Lösung bekannt. Hierbei wird Biomasse mittels Elektroflocculation ionisiert.

DE 197 57 793 A1 beschreibt die Reaktionsorientierung biologischer Reaktionsvorgänge mittels elektromagnetischer Steuersequenzen, insbesondere die synchrone Reaktionsorientierung aller biologischer Reaktionsträgereinheiten, im Besonderen Pilze und Bakterien, im Bioreaktor mittels elektromagnetischer Steuersequenzen, über elektrisch flussorientiert über Ionenbildung, magnetisch, elektrostatisch oder elektromagnetisch wechselnde Felder.US 4,822,470 offenbart ferner ein System und ein Verfahren zur Poration und Fusion von Zellen mittels hochfrequenten elektrischen Pulsen.

DE 35 38 194 A1 beschreibt ein Verfahren zur stoffwechsel- und/oder wachstumssteigernden Behandlung von Mikroorganismen. Hierbei setzt man die Mikroorganismen elektrischen Feldern aus, deren Feldstärke impulsförmig verläuft und deren Spitze 3,5 kV/cm nicht überschreitet.

Somit liegt der Erfindung die Aufgabe zugrunde, eine Vorrichtung zur elektrischen Desintegration anzugeben, welche rasch an sich ändernde Umgebungsbedingungen, insbesondere rasch an geänderte Eigenschaften der organischen Materie anpassbar ist.

Die Erfindung löst die ihr zugrundeliegende Aufgabe bei einer Vorrichtung der eingangs bezeichnet Art durch die Merkmale des Anspruchs 1, insbesondere indem die elektronische Steuereinheit zur Resonanzfrequenzbestimmung eine Regeleinheit mit einem ersten Prozessor aufweist, und zum Ansteuern der Hochspannungsquelle eine Treibereinheit mit einem zweiten Prozessor aufweist, wobei die Treibereinheit dazu eingerichtet ist, zumindest eines der Folgenden zu steuern: Frequenz, Pulsdauer, und Amplitude der Spannung der Hochspannungsquelle, und wobei die Hochspannungsquelle eine Messspule und eine Hochspannungsspule aufweist, welche um denselben Kern gespult sind. Die Erfindung beruht auf der Erkenntnis, dass die Hochspannungsquelle mit dem Elektrodenkörper und der Kammerwand einen Schwingkreis bildet. Sobald sich die Temperatur, Viskosität, der Druck oder Volumenstrom des Fluids in der Kammer ändern, ändert sich auch die Permittivität in der Kammer. Dies beeinflusst wiederum nach allgemein bekannten physikalischen Grundsätzen die Resonanzfrequenz des Schwingkreises. Da eine optimale Felderzeugung auch bei oder zumindest in der Nähe der Resonanzfrequenz gewährleistet ist, hat sich dessen Bestimmung als gut geeignete Maßnahme herausgestellt, auf sich ändernde Bedingungen in der Kammer reagieren zu können.

In einer bevorzugten Weiterbildung der Erfindung ist die Messspule mit der elektronischen Steuereinheit verbunden. Hiermit wird der besondere Vorteil erreicht, dass die Resonanzfrequenz mittels der Messspule bestimmt werden kann, ohne dass Eingriffe in die Kammer selbst notwendig wären.

Vorzugsweise ist die elektronische Steuereinheit dazu eingerichtet, die in der Messspule induzierte Spannung, sowie vorzugsweise die Frequenz der Spannung zu messen, und weiter vorzugsweise eine Anstiegsrate der Spannung zu bestimmen. Aufgrund der Kopplung der Messspule und der Hochspannungsspule über den gemeinsamen Kern ist gewährleistet, dass die Frequenz an der Messspule die gleiche ist wie an der Hochspannungsspule. Zudem nimmt bei Erreichen der Resonanzfrequenz auch die an der Messspule induzierte Spannung ein Maximum ein. Daher ist es mit geringem technischen Aufwand möglich, mittels Überwachung des Spannungsverlaufs an der Messspule festzustellen, ob bzw. wann die Resonanzfrequenz erreicht ist.

Gemäß der Erfindung weist die elektronische Steuereinheit zur Resonanzfrequenzbestimmung eine Regeleinheit mit einem ersten Prozessor, und zum Ansteuern der Hochspannungsquelle eine Treibereinheit mit einem zweiten Prozessor auf, wobei die Treibereinheit dazu eingerichtet ist, zumindest eines der Folgenden zu steuern: Frequenz, Pulsdauer, und Amplitude der Spannung der Hochspannungsquelle. Es hat sich als vorteilhaft herausgestellt, die Auswertung der an der Messspule induzierten Spannung einerseits und das Ansteuern der Hochspannungsquelle andererseits mit zwei dedizierten Prozessoren durchzuführen, weil hierdurch jeweils kleine, wenig Ressourcen beanspruchende Prozessoren verwendet werden können. Die Regeleinheit ist vorzugsweise dazu eingerichtet, in Abhängigkeit der bestimmten Messgrößen der Messspule Steuerbefehle an die Treibereinheit zu übermitteln, um die Frequenz des Gesamtsystems der Resonanzfrequenz anzunähern.

Weiterhin weist die Hochspannungsquelle gemäß der Erfindung die Messspule und die Hochspannungsspule auf, welche um denselben Kern gespult sind. Die Hochspannungsquelle weist vorzugsweise eine Mehrzahl von in Reihe geschalteten Spannungsverdopplern auf, die an die Hochspannungsspule angeschlossen sind.

Vorzugsweise ist die elektronische Steuereinheit dazu eingerichtet, zumindest eine der folgenden Größen in vorbestimmten Zeitabständen automatisch schrittweise zu variieren: Frequenz, Pulsdauer, und Amplitude der Spannung der Hochspannungsquelle. Weiter vorzugsweise ist die elektronische Steuereinheit dazu eingerichtet ist, nach einem ersten erfolgten Variationsschritt einen weiteren Variationsschritt in die gleiche Richtung vorzunehmen, falls die an der Messspule induzierte Spannung nach dem ersten Variationsschritt höher ist als zuvor, und einen Variationsschritt in die entgegengesetzte Richtung vorzunehmen, falls die an der Messspule induzierte Spannung nach dem ersten Variationsschritt geringer ist als zuvor. Hiermit wird ein sich automatisch an geänderte Kammerbedingungen anpassendes System bereitgestellt. Durch das Ablaufen der Variationsschritte wird ständig geprüft, ob eine höhere oder niedrigere Frequenz (oder andere Parameter wie bspw. Pulsweite) an der Messspule zu einer höheren induzierten Spannung führen. Hierbei wird zunächst in einer ersten Richtung verändert, und wenn diese Variation zu einer Verringerung der induzierten Spannung an der Messspule führt, wird in die entgegengesetzte Richtung variiert, so lange, bis die Variation immer im Wechsel um ein Maximum hin und her erfolgt. Dies ist dann der neue optimale Betriebszustand. Optional kann die Spule zwischen 1 und 128 Hz gepulst werden. Dieses geschieht vorzugsweise beim Erreichen des optimalen Betriebszustandes (der optimalen Resonanzfrequenz).

Vorzugsweise ist der Zeitabstand von einem Variationsschritt bis zu einem Variationsschritt in die entgegengesetzte Richtung geringer als der Zeitabstand zwischen zwei Variationsschritten in die gleiche Richtung. Hierdurch wird ein schnelleres Reagieren auf eine Veränderung der Frequenz (oder anderer Parameter wie bspw. der Pulsweite) gewährleistet.

Ferner wird eine Verwendung der Vorrichtung zur elektrischen Desintegration von Zellverbänden offenbart. Demnach werden bei einer solchen Verwendung die Schritte offenbart:
- Bereitstellen einer Kammer, innerhalb welcher ein Elektrodenkörper angeordnet ist,
- Einleiten von Zellverbände enthaltendem Fluid in die Kammer,
- Erzeugen eines elektrischen Feldes in der Kammer derart, dass Zellverbände desintegrieren,
- Ändern des elektrischen Feldes mittels einer mit einer Hochspannungsquelle für den Elektrodenkörper zusammenwirkenden elektronischen Steuereinheit, und
- Bestimmen der Resonanzfrequenz der Hochspannungsquelle.

Hinsichtlich der Vorteile der Verwendung und ihrer nachstehenden bevorzugten Ausführungsformen wird auf die obigen Erläuterungen zur erfindungsgemäßen Vorrichtung verwiesen.

Die Verwendung wird vorzugsweise weitergebildet, indem das Bestimmen der Resonanzfrequenz umfasst:
- Messen der in einer Messspule induzierten Spannung, wobei die Messspule und eine Hochspannungsspule der Hochspannungsquelle um denselben Kern gespult sind.

In einer bevorzugten Ausführungsform der Verwendung umfasst das Ändern des elektrischen Feldes das Steuern von zumindest einem der Folgenden:
- Frequenz,
- Pulsdauer, und
- Amplitude
der Spannung der Hochspannungsquelle.

Gemäß einer weiteren bevorzugten Ausführungsform der Verwendung umfasst diese den Schritt:
- automatisches schrittweises Variieren zumindest einer der folgenden Größen in vorbestimmten Zeitabständen:
- Frequenz,
- Pulsdauer, und
- Amplitude
der Spannung der Hochspannungsquelle.

Die Verwendung wird weitergebildet durch zumindest einen der Schritte:
- Vornehmen eines weiteren Variationsschritts in die gleiche Richtung, falls die an der Messspule induzierte Spannung nach einem ersten Variationsschritt höher ist als zuvor, und
- Vornehmen eines Variationsschrittes in die entgegengesetzte Richtung, falls die an der Messspule induzierte Spannung nach einem ersten Variationsschritt geringer ist als zuvor.

Die Erfindung wird im Folgenden unter Bezugnahme auf die beigefügten Figuren näher beschrieben. Hierbei zeigt:
- Figur 1: eine räumliche Darstellung der erfindungsgemäßen Vorrichtung zur Desintegration von Zellverbänden,
- Figur 2: eine schematische Teildarstellung des funktionalen Aufbaus der erfindungsgemäßen Vorrichtung,
- Figur 3: eine weitere schematische Teildarstellung des funktionalen Aufbaus der erfindungsgemäßen Vorrichtung, und
- Figur 4: eine weitere schematische Teildarstellung des funktionalen Aufbaus der erfindungsgemäßen Vorrichtung.

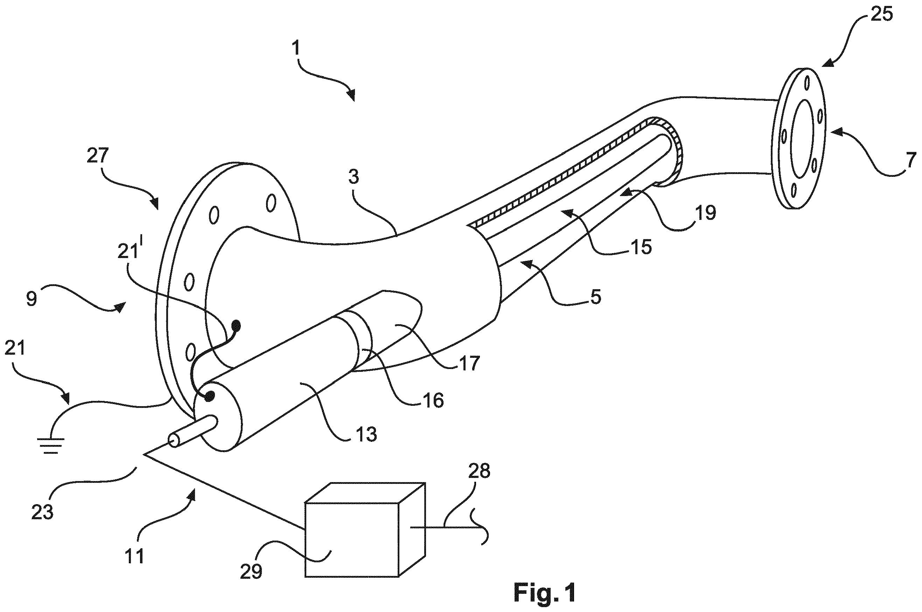

Die in Figur 1 dargestellte Vorrichtung 1 weist ein Gehäuse 3 auf. Das Gehäuse 3 ist abschnittsweise zylindrisch ausgebildet. Innerhalb des Gehäuses 3 ist eine Kammer 5 angeordnet, welche abschnittsweise als Hohlzylinder ausgebildet ist. An zwei gegenüberliegenden Enden des Gehäuses 3 sind ein Einlass 7 zur Aufnahme von Fluid in die Kammer 5 und ein Auslass 9 zur Abgabe von Fluid aus der Kammer 5 angeordnet. Die Vorrichtung 1 weist eine Elektrodeneinheit 11 auf. Die Elektrodeneinheit 11 weist einen Elektrodenkopf 13 und einen Elektrodenkörper 15 auf. Die Elektrodeneinheit 11 ist mittels einer von dem Gehäuse 3 umfassten Elektrodenführung 17 derart aufgenommen, dass sich der Elektrodenkörper 15 innerhalb der Kammer 5 des Gehäuses 3 erstreckt. Die Elektrodenführung 17 ist entsprechend eines Rohrfortsatzes gebildet und definiert eine zentrale Öffnung 16. Der Elektrodenkörper 15 ist vorzugsweise mittels einer (nicht dargestellten) Schraubverbindung mit dem Gehäuse 3 bzw. der Elektrodenführung 17 verbunden. Optional ist der Elektrodenkörper 15 an einer der Elektrodenführung 17 gegenüberliegenden Seite des Gehäuses 3 mit einer weiteren (nicht dargestellten) Elektrodenführung gehaltert. Die Kammer 5 weist eine Wand 19 auf, welche elektrisch von dem Elektrodenkörper 15 isoliert ist. Optional ist die Wand 19 der Kammer 5 abschnittsweise elektrisch isoliert oder mit einem Dielektrikum beschichtet. Das Gehäuse 3 und die Elektrodeneinheit 11 sind mittels einer Erdung 21 geerdet. Optional sind das Gehäuse 3 und der Elektrodenkopf 13 ebenfalls mittels einer Erdung 21' verbunden.

Der Einlass 7 weist einen Flansch 25 zum Anschluss an ein Rohrleitungssystem oder zum Anschluss an eine benachbarte weitere Vorrichtung 1 (nicht dargestellt) auf. Der Auslass 9 weist einen Flansch 27 auf, der ebenfalls zum Anschluss an ein Rohrleitungssystem oder zum Anschluss an eine benachbarte Vorrichtung 1 ausgebildet ist. Durch eine einzelne Vorrichtung 1 oder den Zusammenschluss mehrerer Vorrichtungen 1 mittels der Flansche 25, 27 wird eine Desintegrationseinrichtung gemäß der vorliegenden Erfindung gebildet.

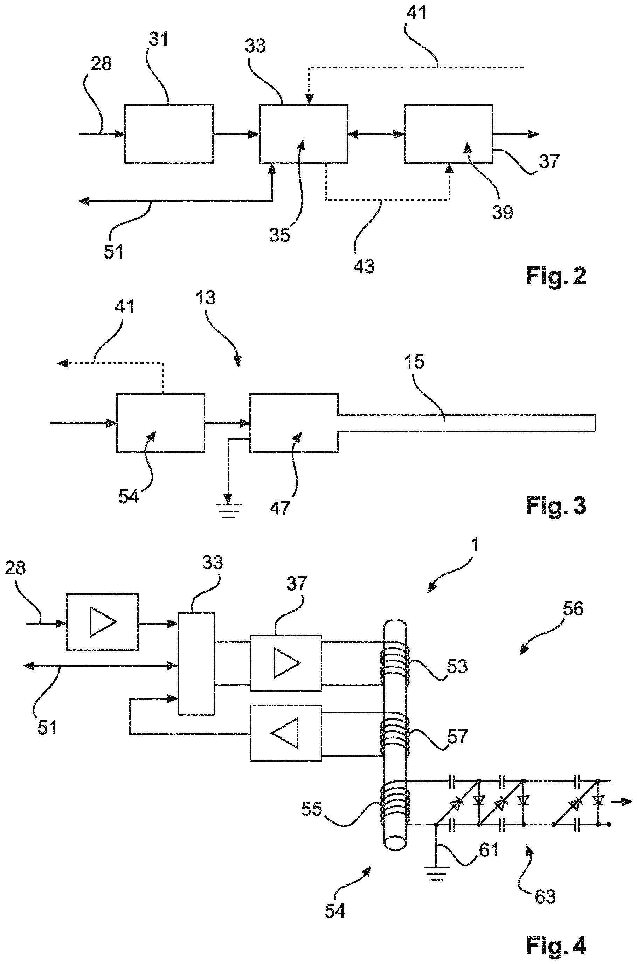

Die Elektrodeneinheit 11 ist dazu ausgebildet, ein elektrisches Feld zwischen dem Elektrodenkörper 15 und der Wand 19 der Kammer 5 auszubilden. Zum Zwecke der Ansteuerung der Elektrodeneinheit 11 weist die Vorrichtung 1 eine elektronische Steuereinheit 29 auf. Diese ist in Figur 2 näher erläutert.

Die elektronische Steuereinheit 29 weist ein Netzteil 31 auf, welches einen Spannungseingang 28 umfasst, der beispielsweise zum Anschluss an eine 230V, 50Hz Wechsel-spannungsquelle eingerichtet ist. Das Netzteil ist verbunden mit einer Regeleinheit 33, die einen ersten Prozessor 35 enthält. Die Regeleinheit 33 ist zur Bestimmung der Resonanzfrequenz des Systems aus Hochspannungsquelle und Kammer/Elektrodenkörper eingerichtet.

Die Regeleinheit 33 ist mittels Signalleitung 43 mit einer Treibereinheit 37 verbunden, welche einen zweiten Prozessor 39 enthält und zur Ansteuerung einer Spuleneinheit 54, welche Teil der Hochspannungsquelle 56 ist, (s. Figur 4) eingerichtet. Eine Datenaustauschleitung 51 ist vorgesehen, um Daten von der Regeleinheit auszulesen, und/oder um diese zu programmieren oder zu steuern.

Im Bereich des Elektrodenkopfes 13 (Figur 3) ist eine Hochspannungskaskade 47 vorgesehen, welche zu einer Vervielfachung der in die Spuleneinheit 54 eingespeisten Spannung führt. Die von der Hochspannungskaskade 47 bereitgestellte Spannung liegt ebenfalls zwischen dem Elektrodenkörper 15 und der Wand 19 an. Die Regeleinheit 33 ist ferner mittels einer Signalleitung 41 mit der Hochspannungsquelle 56 verbunden, um deren Resonanzfrequenz bestimmen zu können. Wie dies vorteilhaft umgesetzt werden kann, ist in Figur 4 veranschaulicht.

Die in Figur 4 gezeigte Spuleneinheit 54 weist eine mit der Treibereinheit 37 verbundene Primärspule 53 mit einer ersten Anzahl Windungen auf. Ferner weist die Spuleneinheit 54 eine Sekundärspule 55 mit einer zweiten Anzahl Windungen auf, vorzugsweise einem Vielfachen der ersten Anzahl Windungen der Primärspule. Schließlich weist die Spuleneinheit 54 eine Messspule 57 auf. Alle Spulen 53, 55, 57 sind um den gleichen Spulenkern gespult, beispielsweise einen Ferritkern. Mittels der Primär- und Sekundärspule 53, 55 wird die Eingangsspannung transformiert. Die in der Messspule 57 induzierte Spannung, und vorzugsweise weitere Größen wie etwa die Frequenz, werden von der Regeleinheit gemessen oder gemessen und an diese übertragen.

Die Sekundärspule ist mit einer Vielzahl von Spannungsverdopplern 63 gekoppelt und mittels Leitung 61 geerdet. Die vervielfachte Spannung liegt zwischen Elektrodenkörper 15 und Wand 19 an.

## Patentansprüche

1. Vorrichtung (1) zur elektrischen Desintegration von Zellverbänden, mit
- einer Elektrodeneinheit (11), welche einen Elektrodenkopf (13) und einen Elektrodenkörper (15) aufweist;
- einer Kammer (5), innerhalb welcher der Elektrodenkörper (15) angeordnet ist, wobei die Kammer (5) eine Wand (19) aufweist, die abschnittsweise oder vollständig elektrisch leitfähig und von dem Elektrodenkörper (15) elektrisch isoliert ist, und wobei die Kammer (5) einen Einlass (7) zur Aufnahme von Zellverbände enthaltendem Fluid aufweist,
- einer in dem Elektrodenkopf (13) angeordneten Hochspannungsquelle (56), die angepasst ist, um mittels Anlegen einer elektrischen Spannung zwischen dem Elektrodenkörper (15) und der Wand (19) ein elektrisches Feld zu erzeugen,
und
- einer elektronischen Steuereinheit (29), die mit der Hochspannungsquelle (56) zusammenwirkt, um das elektrische Feld zu ändern,
**dadurch gekennzeichnet, dass** die elektronische Steuereinheit (29) zur Resonanzfrequenzbestimmung eine Regeleinheit (33) mit einem ersten Prozessor (35) aufweist, und
zum Ansteuern der Hochspannungsquelle (56) eine Treibereinheit (37) mit einem zweiten Prozessor (39) aufweist, wobei die Treibereinheit (37) dazu eingerichtet ist, zumindest eines der Folgenden zu steuern:
- Frequenz,
- Pulsdauer, und
- Amplitude
der Spannung der Hochspannungsquelle (56), und
wobei die Hochspannungsquelle (56) eine mit der elektronischen Steuereinheit (29) verbundene Messspule (57) und eine Hochspannungsspule (55) aufweist, welche um denselben Kern gespult sind.

2. Vorrichtung nach Anspruch 1,
wobei die elektronische Steuereinheit (29) dazu eingerichtet ist, die in der Messspule (57) induzierte Spannung, sowie vorzugsweise die Frequenz der Spannung zu messen, und weiter vorzugsweise eine Anstiegsrate der Spannung zu bestimmen.

3. Vorrichtung nach einem der vorstehenden Ansprüche,
wobei die Hochspannungsquelle eine Mehrzahl von in Reihe geschalteten Spannungsverdopplern (63) aufweist, die an die Hochspannungsspule (55) angeschlossen sind.

4. Vorrichtung nach einem der vorstehenden Ansprüche,
**dadurch gekennzeichnet, dass** die elektronische Steuereinheit (29) dazu eingerichtet ist, zumindest eine der folgenden Größen in vorbestimmten Zeitabständen automatisch schrittweise zu variieren:
- Frequenz,
- Pulsdauer, und
- Amplitude
der Spannung der Hochspannungsquelle.

5. Vorrichtung nach Anspruch 4,
wobei die elektronische Steuereinheit (29) dazu eingerichtet ist, nach einem erfolgten Variationsschritt
- einen weiteren Variationsschritt in die gleiche Richtung vorzunehmen, falls die an der Messspule (57) induzierte Spannung nach dem Variationsschritt höher ist als zuvor, und
- einen Variationsschritt in die entgegengesetzte Richtung vorzunehmen, falls die an der Messspule (57) induzierte Spannung nach dem Variationsschritt geringer ist als zuvor.

6. Vorrichtung nach Anspruch 5,
wobei der Zeitabstand von einem Variationsschritt bis zu einem Variationsschritt in die entgegengesetzte Richtung geringer ist als der Zeitabstand zwischen zwei Variationsschritten in die gleiche Richtung.

7. Vorrichtung nach einem der vorstehenden Ansprüche, wobei die Hochspannungsquelle (56) eine Primärspule (53) aufweist, und wobei die Hochspannungsspule (57), die Primärspule (53) und die Messspule (57) um denselben Kern gespult sind.

## Claims

1. A device (1) for electrically disintegrating cell structures, having
- an electrode unit (11) comprising an electrode head (13) and an electrode body (15);
- a chamber (5) in which the electrode body (15) is disposed, the chamber (5) comprising a wall (19), said wall being electrically conductive in segments or entirely and being electrically insulated from the electrode body (15), and the chamber (5) comprising an inlet (7) for receiving fluid comprising cell structures,
- a high-voltage source (56) disposed in the electrode head (13) and adapted for generating an electrical field by means of applying an electrical voltage between the electrode body (15) and the wall (19),
and
- an electronic control unit (29) interacting with the high-voltage source (56) in order to modify the electrical field,
**characterized in that** the electronic control unit (29) comprises a controller (33) having a first processor (35) for determining a resonant frequency, and
comprises a driver unit (37) having a second processor (39) for actuating the high-voltage source (56), wherein the driver unit (37) is set up for controlling at least one of the following:
- frequency,
- pulse duration, and
- amplitude
of the voltage of the high-voltage source (56), and
wherein the high-voltage source (56) comprises a measurement coil (57) connected to the electronic control unit (29) and a high-voltage coil (56) wound about the same core.

2. The device according to claim 1,
wherein the electronic control unit (29) is set up for measuring the voltage induced in the measurement coil (57) and preferably the frequency of the voltage, and further preferably for determining a rise rate of the voltage.

3. The device according to any one of the preceding claims,
wherein the high-voltage source comprises a plurality of voltage doublers (63) connected in series and connected to the high-voltage coil (55).

4. The device according to any one of the preceding claims,
**characterized in that** the electronic control unit (29) is set up for automatically incrementally varying at least one of the following parameters at predetermined time intervals:
- frequency,
- pulse duration, and
- amplitude
of the voltage of the high-voltage source.

5. The device according to claim 4,
wherein the electronic control unit (29) is set up for, following a completed variation step,
- performing a further variation step in the same direction if the voltage induced at the measurement coil (57) is higher after the variation step than before, and
- performing a further variation step in the opposite direction if the voltage induced at the measurement coil (57) is lower after the variation step than before.

6. The device according to claim 5,
wherein the time interval form one variation step to a variation step in the opposite direction is less that the time interval between two variation steps in the same direction.

7. The device according to any one of the preceding claims, wherein the high-voltage source (56) comprises a primary coil (53), and wherein the high-voltage coil (57), the primary coil (53), and the measurement coil (57) are wound about the same core.

## Revendications

1. Dispositif (1) de désintégration électrique de groupes de cellules, comprenant
- une unité (11) d'électrode, qui a une tête (13) d'électrode et un corps (15) d'électrode ;
- une chambre (5), dans laquelle est disposé le corps (15) de l'électrode, la chambre (15) ayant une paroi (19), qui est conductrice de l'électricité par endroit ou complètement et qui est isolée électriquement du corps (15) de l'électrode, la chambre (5) ayant une entrée (7) de réception de fluide contenant des groupes de cellules,
- une source (56) de haute tension, qui est mise dans la tête (13) de l'électrode et qui est propre à produire un champ électrique, au moyen de l'application d'une tension électrique entre le corps (15) de l'électrode et la paroi (19),
et
- une unité (29) électronique de commande, qui coopère avec la source (56) de haute tension, afin de faire varier le champ électrique,
**caractérisé en ce que** l'unité (29) électronique de commande a, pour la détermination de la fréquence de résonnance, une unité (33) de régulation ayant un premier processeur (35), et,
pour commander la source (56) de haute tension, a une unité (37) d'attaque ayant un deuxième processeur (39), l'unité (37) d'attaque étant conçue pour commander au moins l'une de ce qui suit :
- la fréquence,
- la durée d'impulsion, et
- l'amplitude
de la tension de la source (56) de haute tension, et
dans lequel la source (56) de haute tension a une bobine (57) de mesure reliée à l'unité (29) électronique de commande et une bobine (55) de haute tension, qui sont enroulées autour du même noyau.

2. Dispositif suivant la revendication 1,
dans lequel l'unité (29) électronique de commande est conçue pour mesurer la tension induite dans la bobine (57) de mesure, ainsi que, de préférence, la fréquence de la tension, et pour déterminer, en outre, de préférence, une vitesse d'élévation de la tension.

3. Dispositif suivant l'une des revendications précédentes,
dans lequel la source de haute tension a une pluralité de doubleurs (63) de tension montés en série, qui sont connectés à la bobine (55) de haute tension.

4. Dispositif suivant l'une des revendications précédentes,
**caractérisé en ce que** l'unité (29) électronique de commande est conçue pour faire varier, par palier automatiquement, au moins l'une des grandeurs suivantes à des intervalles de temps déterminés à l'avance :
- la fréquence,
- la durée d'impulsion, et
- l'amplitude
de la tension de la source de haute tension.

5. Dispositif suivant la revendicaton 4,
dans lequel l'unité (29) électronique de commande est conçue pour, après qu'un stade de variation a été effectué,
- effectuer un autre stade de variation dans le même sens, si la tension induite sur la bobine (57) de mesure est plus haute après le stade de variation qu'auparavant, et
- effectuer un stade de variation dans le sens contraire, si la tension induite sur la bobine (57) de mesure est plus basse après le stade de variation qu'auparavant.

6. Dispositif suivant la revendication 5,
dans lequel le laps de temps d'un stade de variation à un autre stade de variation dans le sens contraire est plus petit que le laps de temps entre deux stades de variation dans le même sens.

7. Dispositif suivant l'une des revendications précédentes, dans lequel la source (56) de haute tension a une bobine (53) primaire et dans lequel la bobine (57) de haute tension, la bobine (53) primaire et la bobine (57) de mesure sont enroulées autour du même noyau.
